# EUROPÄISCHE PATENTSCHRIFT

(11) **EP 0 440 906 B1**
(45) Veröffentlichungstag und Bekanntmachung des Hinweises auf die Patenterteilung: **11.08.1993**
(21) Anmeldenummer: 90122930.2
(22) Anmeldetag: 30.11.1990
(51) Int. Cl.: G01N 33/00

(54) **Schaltungsanordnung für ein Gasmessgerät**
Circuit arrangement for a gas sensor
Circuit pour un capteur de gaz

(30) Priorität: 03.02.1990 DE 4003244
(43) Veröffentlichungstag der Anmeldung: 14.08.1991
(73) Patentinhaber: AUERGESELLSCHAFT GMBH, D-12059 Berlin (DE)
(72) Erfinder: Höht, Wolfgang, W-1000 Berlin 47 (DE); Kraatz, Heinz, W-1000 Berlin 44 (DE); Mähl, Dieter, Dr., W-1000 Berlin 20 (DE)

(56) Entgegenhaltungen:
- EP-A- 0 234 251
- DE-A- 2 714 040
- GB-A- 1 447 488

## Beschreibung

Die Erfindung betrifft eine Schaltungsanordnung für ein Gasmeßgerät zur Messung und Anzeige von Konzentrationen von in Luft enthaltenen brennbaren Gasen und Dämpfen nach dem Oberbegriff des Anspruchs 1.

Eine derartige Schaltungsanordnung ist beispielsweise in der DE-PS 27 14 040 beschrieben. Bei dieser bekannten Schaltungsanordnung werden zur Messung nach dem Wärmeleitfähigkeitsprinzip und nach dem Wärmetönungsprinzip jeweils eine Meßbrücke verwendet, die über eine Ansteuerschaltung und zwei Schwellwertschalter miteinander verbunden sind, wobei die letzteren je nach eingestellter Gaskonzentration entweder auf die Meßbrücke nach dem Wärmeleitfähigkeitsprinzip oder auf die Meßbrücke nach dem Wärmetönungsprinzip das Meßsystem umschalten. Für die Meßbrücke nach dem Wärmeleitfähigkeitsprinzip besteht diese aus zwei Sensoren, von denen ein Sensor dem Meßgas ausgesetzt ist, während der andere Sensor gleicher Bauart in einer vom Meßgas abgeschlossenen Kammer untergebracht, nur auf die Umgebungstemperatur reagiert. Auch die Meßbrücke nach dem Wärmetönungsprinzip besteht aus zwei Sensoren, von denen der eine katalytisch aktiv ist und ebenfalls dem Meßgas ausgesetzt ist, und der andere dient als katalytisch inaktives Vergleichselement zur Kompensation der übrigen Umwelteinflüsse. Hieraus wird deutlich, daß die beiden in einer Meßschaltung zusammengeschalteten Meßsysteme zwei Meßbrücken mit vier Sensoren aufweist. Dies ist einmal schaltungstechnisch und zum anderen im Hinblick auf die Verwendung von vier Meßsensoren in einem Meßkopf kostenintensiv und baulich aufwendig. Darüber hinaus erfolgt die Umschaltung von einem System auf das andere mit einer entsprechenden Verzögerung, die sich dadurch ergibt, daß durch die jeweilige Umschaltung auf das andere System, dieses erneut aktiviert und die Meßsensoren in ein thermisches Gleichgewicht gebracht werden müssen, um meßbereit zu sein.

Der Erfindung liegt daher die Aufgabe zugrunde, eine Schaltungsanordnung der eingangs genannten Art zu entwickeln, die mit einfachen schaltungstechnischen Mitteln eine eindeutige Messung des brennbaren Gases in Luft sowohl in dem niedrigen Konzentrationsbereich nach dem Wärmetönungsprinzip als auch in dem hohen Konzentrationsbereich nach dem Wärmeleitungsprinzip gewährleistet, und zwar bei verzögerungsfreier Umschaltung von einem System auf das andere.

Diese Aufgabe wird durch die Schaltungsanordnung gemäß Patentanspruch 1 gelöst.

Die mit der Erfindung erzielten Vorteile bestehen insbesondere darin, daß für beide Meßprinzipien, nämlich für das Wärmetönungsverfahren und das Wärmeleitungsverfahren die Anzahl der Meßsensoren von bisher vier Sensoren auf jetzt 3 Sensoren bzw. 2 Sensoren reduziert werden kann. Darüber hinaus wird der katalytisch aktive Meßsensor bei für ihn schädlichen hohen Gaskonzentrationen, die stets zu einer Vergiftung der katalytisch aktiven Oberfläche und somit zu einer vorzeitigen Zerstörung des Sensors führen, abgeschaltet und geschont.

Weitere vorteilhafte Weiterentwicklungen sind in den abhängigen Ansprüchen 2 bis 9 gekennzeichnet.

Ausführungsbeispiele der Erfindung sind in der Zeichnung dargestellt und werden im folgenden näher beschrieben.
Es zeigen:
- Fig. 1: eine erfindungsgemäße Meßbrückenanordnung zur Wärmetönungs-und Wärmeleitungsmessung, wobei die Meßbrücke für die Wärmetönungsmessung eingeschaltet ist,
- Fig. 2: die Meßbrückenanordnung nach Fig. 1, wobei die Meßbrücke für die Wärmeleitungsmessung eingeschaltet ist, und
- Fig. 3 und 4: zwei weitere Ausführungsbeispiele nach den Figuren 1 und 2, wobei anstelle des an die Meßbrücke angeschalteten inaktiven Sensors ein ohm'scher Ersatzwiderstand verwendet wird.

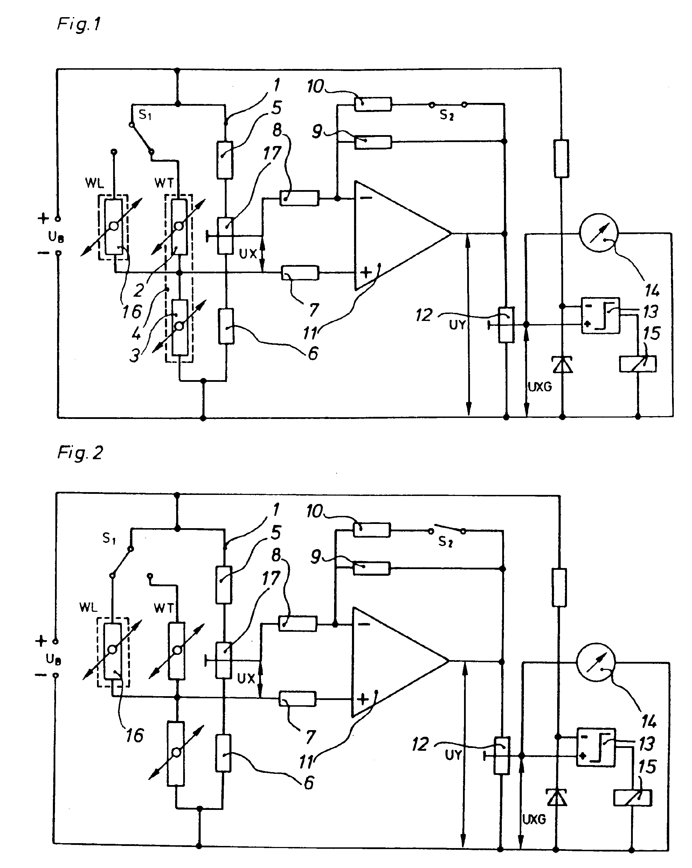

Wie aus Fig. 1 ersichtlich ist, besteht die Meßbrückenanordnung zur Messung nach dem Wärmetönungsprinzip im wesentlichen aus einer Meßbrücke 1 mit den Meßsensoren 2 und 3 in dem einen Brückenzweig, wobei der Meßsensor 2 als aktives, d. h. als gasempfindliches Element wirkt, während der Meßsensor 3 als inaktives Vergleichselement zur Kompensation der übrigen Umwelteinflüsse, unabhängig von der Gaskonzentration dient. Die Meßsensoren 2 und 3 befinden sich in einer gemeinsamen druckfesten Meßkammer 4, die den Meßkopf darstellt, in dem das zu messende Luft-Gas-Gemisch hineingeleitet wird. In dem anderen Brückenzweig sind Widerstände 5 und 6 sowie ein Potentiometer 17 angeordnet, mit dem die Meßbrücke an gasfreier Luft (ohne Meßgas) abgeglichen wird, und zwar auf einen Spannungswert UX von Null mV. Das ist der sogenannte Nullabgleich. Die bei der Messung mit der Meßbrücke 1 in der Brückendiagonalen entstehende Brückenspannung, die gleichbedeutend mit der Signalspannung ist, wird über die Widerstände 7 und 8 einem mit Gegenkopplungswiderständen 9 und 10 betriebenen Verstärker 11 zugeführt. Das Signal wird am Verstärker 11 im Verhältnis der Gegenkoppelungswiderstände 9 und 10 und dem Widerstand 8 auf eine Signalausgangsspannung UY verstärkt. Am Ausgang des Verstärkers 11 liegt über ein Potentiometer 12 ein als Komparator geschalteter Operationsverstärker 13, dessen Schaltschwelle mittels des Potentiometers 12 eingestellt werden kann. Mit dem Potentiometer 12 wird außerdem für die konzentrations-proportionale Anzeige ein Anzeigeinstrument 14 eingestellt. Die am Operationsverstärker 13 eingestellte Schaltschwelle dient zum Umschalten von der Wärmetönungsmessung, die bei Vorhandensein von geringen Gaskonzentrationen angewendet wird, auf die Wärmeleitfähigkeitsmessung bei hohen Gaskonzentrationen. Hierbei wird bei der Wärmetönungsmessung im Bereich der jeweiligen unteren Explosionsgrenze (UEG) des zu messenden Stoffes und bei der Wärmeleitfähigkeitsmessung im Bereich der oberen Explosionsgrenze (OEG) gemessen. Der Ausgang des Operationsverstärkers 13 steuert einen elektronischen Schalter 15, der ein Relais sein kann, das die Relaiskontakte S₁ und S₂ aufweist, wobei der Kontakt S₁ in dem Brückenzweig mit den Meßsensoren 2 und 3 geschaltet ist und der Kontakt S₂ in der Verstärkerschaltung des Verstärkers 11 hinter dem Gegenkopplungswiderstand 10. An dem Diagonalzweig der Meßbrücke 1 ist in Reihe mit dem inaktiven Meßsensor 3 ein außerhalb des Meßkopfes 4 vorgesehener inaktiver Meßsensor 16 geschaltet. Der inaktive Meßsensor 16 dient im Zusammenhang mit dem Meßsensor 3 zur Messung nach dem Wärmeleitungsprinzip. Der am Operationsverstärker 13 eingestellte Schwellwert als Umschaltschwelle von der Messung nach dem Wärmetönungsprinzip auf das Wärmeleitungsprinzip ist vorzugsweise auf einen Wert zwischen 120 % bis 150 % des vorgesehenen Meßbereichs einzustellen.

Bei einem vorgesehenen Meßbereich von 0 bis 5 Vol-% für die Messung von beispielsweise Methan, bedeutet dies, daß der am Operationsverstärker 13 eingestellte Schwellwert zwischen 6 Vol-% und 7,5 Vol-% liegt und bei Erreichung dieses Wertes die Umschaltung von der Wärmetönungsmessung auf die Wärmeleitungsmessung erfolgt. Durch diese gezielte Umschaltung in den genannten Konzentrationsbereichen wird der katalytisch aktive Meßsensor 2 aus der Meßbrücke 1 herausgeschaltet, so daß die höheren Gaskonzentrationen von dem Meßsensor 2 ferngehalten werden. Der Meßsensor 2 wird geschont. Es werden Kohlenstoffabscheidungen auf der aktiven Oberfläche des Sensors und eine damit verbundene Empfindlichkeitsminderung bzw. Zerstörung vermieden. Die Umschaltung von der Wärmetönungsmessung auf die Wärmeleitungsmessung erfolgt derart, daß der Relaiskontakt S₁ den aktiven Meßsensor 2 aus der Meßbrücke 1 ausschaltet und den Meßsensor 16 in die Brücke einschaltet. Die Umschaltung erfolgt verzögerungsfrei, da der inaktive Meßsensor 3 von der vorangegangenen Wärmetönungsmessung her aktiviert ist und sich bereits im thermischen Gleichgewicht befindet.
Der Sensor ist sofort meßbereit.
Die Schaltungsanordnung funktioniert folgendermaßen: In Fig. 1 ist das Prinzip der Wärmetönungsmessung dargestellt, in dem der Relaiskontakt S₁ des Relais 15 in der Meßbrücke 1 an den aktiven Meßsensor 2 geschaltet und der Relaiskontakt S₂ den Verstärkerschaltkreis mit dem Verstärker 11 und dem Gegenkopplungswiderstand 10 geschlossen hält. Die Meßbrücke 1 wird an gasfreier Luft mit einem Potentiometer 17 auf einen Spannungswert UX gleich 0,0 mV abgeglichen. Bei Gasaufgabe auf den Meßkopf bzw. auf die Meßkammer 4 wird an dem aktiven Meßsensor 2 das Gas verbrannt. Hierbei erhöht sich der Widerstand des Meßsensors 2 durch die Temperaturerhöhung und führt zu einer Wärmetönung, während der inaktive Meßsensor 3 seinen Widerstand verringert und eine Wärmeleitung bewirkt. Diese Widerstandsänderungen erzeugen an der Brückendiagonalen ein der Konzentration linearproportiales Signal UXG. Dieses Signal wird durch den nachgeschalteten Verstärker 11 im Verhältnis der Gegenkopplungswiderstände 9 und 10 zum Widerstand 8 auf das Signal UYG verstärkt. Mit dem Potentiometer 12 werden zwei Schaltschwellen eingestellt, und zwar einmal für die konzentrations-proportionale Anzeige am Anzeigeinstrument 14 und zum anderen die für den Operationsverstärker 13. Die Anzeige kann in der einfachsten Ausführung durch ein Analog-Instrument erfolgen.

In Fig. 2 ist das Prinzip der Wärmeleitungsmessung dargestellt. Erreicht und überschreitet das Signal UYₛ den für die Wärmetönungsmessung vorgesehenen Bereich, der am Operationsverstärker 13 eingestellt ist, dann wird das Relais 15 angesteuert und schaltet mittels des Relaiskontaktes S₁ verzögerungsfrei vom aktiven Meßsensor 2 auf den außerhalb der Meßkammer 4 angeordneten inaktiven Meßsensor 16. Damit wird der letztere in die Meßbrücke 1 eingeschaltet, während der Meßsensor 2 aus der Brücke herausgeschaltet wird. Gleichzeitig schaltet der Schaltkontakt S₂ des Relais 15 den Gegenkopplungswiderstand 10 ab, wodurch der Verstärker 11 eine derartige Verstärkung erfährt, daß die Schaltschwelle des Operationsverstärkers 13 alleine durch das Wärmeleitungssignal des inaktiven Meßsensors 3 gehalten wird. Auf diese Weise ist die Schaltungsanordnung bis zu Gaskonzentrationen von 100 % des jeweiligen zu messenden Gases in ihrer Anzeige einsinnig-eindeutig. Dies bedeutet, daß die Eindeutigkeit des Meßergebnisses auch bei der Messung von hohen Konzentrationen an brennbaren Anteilen gewährleistet ist.
Bei Unterschreiten der Gaskonzentration unter den für die Wärmeleitungsmessung vorgesehenen Bereich schaltet der Operationsverstärker 13 automatisch wieder auf die Wärmetönungsmessung nach Fig. 1 um.

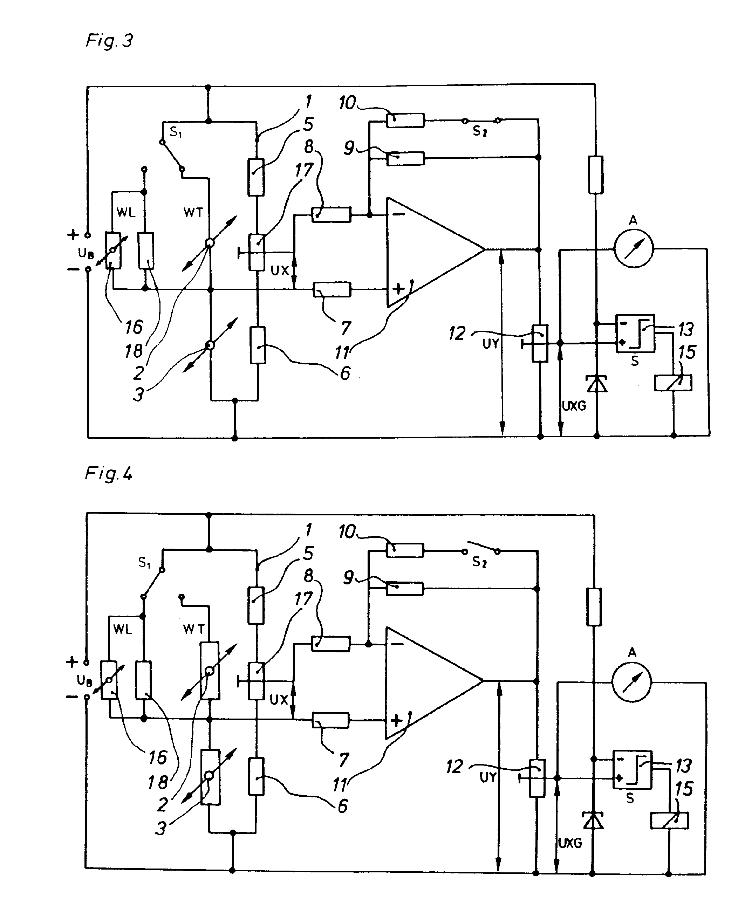

Wie aus den Figuren 3 und 4 ersichtlich ist, kann anstelle des an den Diagonalzweig der Meßbrücke 1 angeschalteten inaktiven Meßsensors 16 vorteilhaft ein ohm'scher Ersatzwiderstand 18 verwendet werden.

## Patentansprüche

1. Schaltungsanordnung für ein Gasmeßgerät zur Messung und Anzeige von in Luft enthaltenen brennbaren Gasen und Dämpfen mit zwei einstellbaren Meßsystemen, einem System für eine Messung mit einer Meßbrücke nach dem Wärmeleitfähigkeitsprinzip und einem System für eine Messung mit einer weiteren Meßbrücke nach dem Wärmetönungsprinzip, dadurch gekennzeichnet, daß
a) eine Meßbrücke (1) für beide Meßsysteme vorgesehen ist, wobei in den einen Brückenzweig ein katalytisch aktiver Meßsensor (2) und ein katalytisch inaktiver Meßsensor (3) als Vergleichselement für das Wärmetönungsprinzip geschaltet ist, während an den Diagonalzweig ein weiterer inaktiver Meßsensor (16) für das Wärmeleitfähigkeitsprinzip geschaltet ist, und
b) die Umschaltung von einem Meßsystem auf das andere mittels eines Schwellwertschalters (13) und einem diesen nachgeschalteten elektronischen Schalter (15) derart erfolgt, daß dieser entweder den weiteren inaktiven Meßsensor (16) in den einen Zweig der Meßbrücke (1) in Reihe mit dem katalytisch inaktiven Meßsensor (3) einschaltet und den katalytisch aktiven Meßsensor (2) herausschaltet oder umgekehrt.

2. Schaltungsanordnung nach Anspruch 1, dadurch gekennzeichnet, daß der Schwellwertschalter (13) als Komparator oder als Operationsververstärker geschaltet ist und eine Schaltschwelle aufweist, die dem Umschaltwert vom Wärmetönungs- auf das Wärmeleitfähigkeitsprinzip entspricht.

3. Schaltungsanordnung nach Anspruch 1, dadurch gekennzeichnet, daß in den Diagonalzweig der Meßbrücke (1) über Widerstände (7, 8) ein mit zwei parallelgeschalteten Gegenkopplungswiderständen (9, 10) betriebener Verstärker (11) geschaltet ist, dessen Ausgang über ein Potentiometer (12) an den Schwellwertschalter (13) geschaltet ist, der den elektronischen Schalter (15) steuert.

4. Schaltungsanordnung nach Anspruch 1 bis 3, dadurch gekennzeichnet, daß der elektronische Schalter (15) ein Reed-Relais ist, mit einem Relaiskontakt (S₁), der von dem Brückenzweig mit dem aktiven Meßsensor (2) auf den Diagonalzweig mit dem weiteren inaktiven Meßsensor (16), und umgekehrt, schaltbar ist.

5. Schaltungsanordnung nach Anspruch 4, dadurch gekennzeichnet, daß ein weiterer Relaiskontakt (S₂) des Reed-Relais (15) in die Verstärkerschaltung des Verstärkers (11) hinter einen der Gegenkopplungswiderstände (10) geschaltet ist.

6. Schaltungsanordnung nach Anspruch 1 bis 5, dadurch gekennzeichnet, daß der am Schwellwertschalter (13) einstellbare Schwellwert als Umschaltschwelle von der Messung nach dem Wärmetönungsprinzip auf das Wärmeleitfähigkeitsprinzip vorzugsweise einen Wert zwischen 120 % bis 150 % des vorgesehenen Meßbereichs aufweist.

7. Schaltungsanordnung nach Anspruch 1, dadurch gekennzeichnet, daß der an den Diagonalzweig der Meßbrücke (1) angeschaltete inaktive Meßsensor (16) ein ohm'scher Ersatzwiderstand ist.

8. Schaltungsanordnung nach Anspruch 1, dadurch gekennzeichnet, daß der katalytisch aktive - und katalytisch inaktive Meßsensor (2, 3) des Brückenzweiges in einen gemeinsamen druckfesten Meßkopf (4) angeordnet sind.

9. Schaltungsanordnung nach Anspruch 8, dadurch gekennzeichnet, daß der an den Diagonalzweig der Meßbrücke (1) angeschaltete inaktive Meßsensor (16) in einem eigenen Gehäuse angeordnet und kein Bestandteil des Meßkopfes ist.

## Claims

1. Circuit arrangement for a gas measuring apparatus for measuring and indicating combustible gases and vapours held in air with two adjustable measuring systems, one system for a measurement with a bridge in accordance with the thermal conductivity principle and one system for a measurement with a further measuring bridge in accordance with the heat tone principle, characterised in that
a) a measuring bridge (1) is provided for both measuring systems, whereby in the one bridge branch is connected a catalytically active measuring sensor (2) and a catalytically inactive measuring sensor (3) as a comparative element for the heat tone principle; whilst on the diagonal branch a further inactive measuring sensor (16) is connected for the thermal conductivity principle, and
b) the switching over from one measuring system to the other by means of a threshold value switch (13) and an electronic switch (15) connected on the load side thereof takes place in such a way that the latter either switches in the further inactive measuring sensor (16) into the one branch of the bridge (1) in series with the catalytically inactive measuring sensor (3) and switches out the catalytically active measuring sensor (2) or vice versa.

2. Circuit arrangement according to claim 1, characterised in that the threshold value switch (13) is connected as a comparator or as an operational amplifier and has a switching threshold which corresponds to the switch over value from the heat tone principle to the thermal conductivity principle.

3. Circuit arrangement according to claim 1, characterised in that in the diagonal branch of the measuring bridge (1) above resistances (7,8) is connected an amplifier (11) operated by two parallel connected feedback resistors (9,10) the output from which amplifier (11) is connected via a potentiometer (12) to the threshold value switch (13) which controls the electronic switch (15).

4. Circuit arrangement according to claims 1 to 3, characterised in that the electronic switch (15) is a reed relay with a relay contact (S₁) which can be switched from the bridge branch with the active measuring sensor (2) to the diagonal branch with the further inactive measuring sensor (16) and vice versa.

5. Circuit arrangement according to claim 4, characterised in that a further relay contact (S₂) of the reed relay (15) is connected in the amplifier circuit of the amplifier (11) behind one of the feedback resistors (10).

6. Circuit arrangement according to claims 1 to 5, characterised in that the threshold value adjustable on the threshold value switch (13) as a switch over threshold from measuring in accordance with the heat tone principle to the thermal conductivity principle preferably has a value between 120% to 150% of the measuring range provided.

7. Circuit arrangement according to claim 1, characterised in that the inactive measuring sensor (16) connected to the diagonal branch of the measuring bridge (1) is an Ohm's equivalent resistance.

8. Circuit arrangement according to claim 1, characterised in that the catalytically active and catalytically inactive measuring sensors (2,3) of the bridge branch are arranged in a common pressure-proof measuring head (4).

9. Circuit arrangement according to claim 8, characterised in that the inactive measuring sensor (16) connected to the diagonal branch of the measuring bridge (1) is arranged in its own housing and is not a component of the measuring head.

## Revendications

1. Circuit pour un capteur de gaz destiné à mesurer et afficher des vapeurs et gaz combustibles contenus dans l'air, avec deux systèmes de mesures réglables, un système pour une mesure avec un pont de mesure utilisant la conduction de chaleur, et un système pour une mesure avec un autre pont de mesure utilisant la chaleur de réaction,
**caractérisé** en ce que
a) un pont de mesure (1) est prévu pour les deux systèmes de mesure, un capteur de mesure catalytiquement actif (2) et un capteur de mesure catalytiquement inactif (3), comme élément comparateur, étant reliés dans une première branche du pont pour l'utilisation de la chaleur de réaction, tandis qu'un capteur de mesure inactif supplémentaire (16) est relié à la branche diagonale pour l'utilisation de la conduction de chaleur, et
b) le passage d'un système de mesure à l'autre s'effectue au moyen d'un commutateur à valeur de seuil (13), suivi d'un commutateur électronique (15), de telle sorte que ce commutateur (15), soit connecte le capteur de mesure inactif supplémentaire (16) dans ladite première branche du pont de mesure (1), en série avec le capteur de mesure catalytiquement inactif (3), et déconnecte le capteur de mesure catalytiquement actif (2), soit effectue l'inverse.

2. Circuit selon la revendication 1, **caractérisé** en ce que le commutateur à valeur de seuil (13) est relié en comparateur ou en amplificateur opérationnel, et il présente un seuil de commutation qui correspond à la valeur de passage de l'utilisation de la chaleur de réaction à l'utilisation de la conduction de chaleur.

3. Circuit selon la revendication 1, **caractérisé** en ce qu'un amplificateur (11), exploité avec deux résistances de contre-réaction (9, 10) montées en parallèle, est relié dans la branche diagonale du pont de mesure (1) par l'intermédiaire de résistances (7, 8), et sa sortie est reliée par l'intermédiaire d'un potentiomètre (12) au commutateur à valeur de seuil (13), qui commande le commutateur électronique (15).

4. Circuit selon l'une quelconque des revendications 1 à 3, **caractérisé** en ce que le commutateur électronique (15) est un relais reed, avec un contact de relais (S₁) qui peut être commuté de la branche du pont présentant le capteur de mesure actif (2) sur la branche diagonale présentant le capteur de mesure inactif supplémentaire (16), et vice-versa.

5. Circuit selon la revendication 4, **caractérisé** en ce qu'un autre contact de relais (S₂) du relais reed (15) est relié dans le circuit d'amplification de l'amplificateur (11), à la suite d'une des résistances de contre-réaction (10).

6. Circuit selon l'une quelconque des revendications 1 à 5, **caractérisé** en ce que la valeur de seuil pouvant être réglée au commutateur à valeur de seuil (13) présente de préférence, comme seuil de passage de la mesure utilisant la chaleur de réaction à la mesure utilisant la conduction de chaleur, une valeur comprise entre 120 % et 150 % de la plage de mesure prévue.

7. Circuit selon la revendication 1, **caractérisé** en ce que le capteur de mesure inactif (16), relié à la branche diagonale du pont de mesure (1), est une résistance ohmique équivalente.

8. Circuit selon la revendication 1, **caractérisé** en ce que le capteur de mesure catalytiquement actif et le capteur de mesure catalytiquement inactif (2, 3) de la branche du pont sont disposés dans une tête de mesure commune (4), résistante à la pression.

9. Circuit selon la revendication 8, **caractérisé** en ce que le capteur de mesure inactif (16), relié à la branche diagonale du pont de mesure (1), est disposé dans un propre boîtier et ne fait pas partie de la tête de mesure.
